# FASCICULE DE BREVET EUROPEEN

(11) **EP 0 466 538 B1**
(45) Date de publication et mention de la délivrance du brevet: **15.02.1995**
(21) Numéro de dépôt: 91401665.4
(22) Date de dépôt: 20.06.1991
(51) Int. Cl.: A61F 5/01

(54) **Articulation pour orthèse des membres inférieurs verrouillable en position d'extension de ces membres**
In der Gliedmassenstreckstellung verriegelbares Gelenk für eine Untergliedmassenorthese
Joint for lower limbs orthosis, lockable in the stretched position of these limbs

(30) Priorité: 10.07.1990 FR 9008760
(43) Date de publication de la demande: 15.01.1992
(73) Titulaire: ETABLISSEMENTS PROTEOR Société anonyme dite:, 21100 Dijon (FR)
(72) Inventeur: Vera, Bernard, F-21250 Seure (FR)
(74) Mandataire: Jolly, Jean-Pierre

(56) Documents cités:
- EP-A- 0 016 268
- AU-A- 480 049
- FR-A- 517 988
- FR-A- 743 791
- FR-A- 1 588 857
- FR-A- 2 566 850
- US-A- 2 433 571
- US-A- 2 632 440
- US-A- 4 353 361

## Description

La présente invention concerne une articulation pour orthèse des membres inférieurs, qui peut être verrouillée en position d'extension de ces membres.

Pour plus de clarté, on se réfèrera ci-après à une articulation du genou, mais il apparaîtra clairement à l'homme de l'art que l'invention s'applique également aux articulations de la cheville ou de la hanche.

On sait que certaines orthèses pour handicapés des membres inférieurs doivent comporter une articulation que l'on puisse verrouiller en position d'extension des membres pour permettre au patient de se déplacer en s'aidant de cannes, et que le patient puisse lui-même déverrouiller, pour amener l'orthèse en position de flexion, afin de lui donner la possibilité de s'asseoir. De telles articulations doivent cependant pouvoir être utilisées parfois en articulation libre, dans le cas où le patient dispose encore d'une certaine liberté de mouvement.

De telles orthèses comportent en général deux montants inférieurs et deux montants supérieurs, ou "attelles", réunis transversalement par des entretoises incurvées ou "embrasses", qui épousent la forme transversale du membre appareillé. Une articulation est prévue entre chacune des attelles inférieure et supérieure et chaque articulation comporte deux branches articulées entre elles autour d'un axe de rotation et destinées à être rendues solidaires, respectivement, du montant inférieur et du montant supérieur associés. Ces branches peuvent avoir la forme de fourreaux, dans lesquels vient se loger l'extrémité associée des montants, qui en est ensuite rendue rigidement solidaire par des vis et éventuellement des écrous. Inversement, l'extrémité des montants contiguë aux dites branches peut former un fourreau dans lequel vient se loger, avant d'en être rendue solidaire, la branche associée de l'articulation.

Il va de soi que les systèmes de verrouillage de ces articulations doivent être peu volumineux, fiables et faciles à manoeuvrer par le patient lui-même.

L'invention vise à proposer un dispositif de verrouillage à commande manuelle répondant à ces impératifs.

L'invention vise également à proposer un dispositif de ce type dans lequel l'articulation de la prothèse passe automatiquement en position de verrouillage, lorsque le patient passe de la position de flexion des membres inférieurs à la position d'extension, par exemple lorsqu'il se redresse à partir d'une position assise.

AU-A-480 049 décrit un système de verrouillage pour une articulation d'orthèse du type mentionné ci-dessus, dans lequel un disque solidaire d'une branche de l'articulation comporte une échancrure qui coopère avec un levier monté pivotant autour d'un axe solidaire d'une autre branche de l'articulation, à l'encontre d'un moyen de rappel élastique, une partie en saillie de ce levier pouvant venir s'engager dans l'échancrure du disque.

Ce système de verrouillage est à simple effet, alors qu'il serait souhaitable de parvenir à bloquer le verrou en deux positions (ouverture permanente ou fermeture permanente).

L'invention vise à proposer un tel dispositif qui, par la suppression d'une simple butée, permette de transformer l'articulation en articulation libre, pour un patient disposant encore partiellement de ses mouvements.

A cet effet, l'invention a pour objet une articulation pour orthèse des membres inférieurs, à deux branches articulées entre elles par un axe de rotation, comportant un système de verrouillage comprenant une gâchette montée pivotante autour d'un axe par rapport à une première branche de l'articulation et actionnable manuellement par une partie faisant saillie à l'extérieur de cette première branche, cette gâchette coopérant avec un profil ménagé sur la seconde branche de l'articulation et étant sollicitée par un organe de rappel élastique qui prend appui, à une extrémité, contre une partie de la première branche, tandis que son autre extrémité prend appui contre une partie de la gâchette apte à se déplacer, cette articulation étant caractérisée en ce que le profil ménagé sur la seconde branche de l'articulation est un profil de came tel que, lorsque la gâchette pivote autour de son axe, la partie de cette gâchette se déplace, entre cet axe et le point d'appui de l'organe de rappel élastique sur la première branche, pour passer d'une première position dans laquelle le profil interdit à la gâchette non sollicitée manuellement de se déplacer et verrouille en position les deux branches de la gâchette l'une par rapport à l'autre, à une seconde position dans laquelle la gâchette sollicitée manuellement pivote autour de son axe de rotation pour échapper au profil de came et venir en butée contre un organe de butée amovible solidaire de la première branche, les positions relatives de l'organe de butée, de l'axe de rotation de la gâchette et des points d'appui de l'organe de rappel élastique sur cette gâchette et sur la première branche étant telles que, lorsque la gâchette est sollicitée manuellement, le point d'appui sur la gâchette de l'organe de rappel élastique, au cours du mouvement de la gâchette, ne traverse pas la droite réunissant l'axe de rotation de la gâchette au point d'appui de l'organe de rappel contre une partie de la première branche de l'articulation, de manière que la gâchette tende à revenir à sa position initiale lorsqu'elle n'est plus sollicitée manuellement.

Avantageusement, la gâchette, son axe de rotation et l'organe de rappel élastique seront logés dans un évidement de la première branche et, à travers une lumière ménagée suivant la tranche de celle-ci, une partie de la gâchette formant organe manuel de commande fera saillie à l'extérieur.

De préférence, l'organe de butée est fixé de façon amovible sur la première branche, de manière à pouvoir être séparé de celle-ci. Dans cette éventualité, lorsque la gâchette sera sollicitée manuellement, le point d'appui sur la gâchette de l'organe de rappel élastique pourra traverser la droite réunissant l'axe de rotation de la gâchette au point d'appui de l'organe de rappel contre une partie de la première branche de l'articulation. La gâchette n'aura plus alors tendance à revenir à sa position initiale sous la sollicitation de l'organe de rappel élastique et l'on pourra alors utiliser l'articulation comme une articulation libre.

L'organe de butée pourra être constitué simplement par une goupille traversant le logement ménagé pour la gâchette dans la première branche.

L'organe de rappel élastique pourra par exemple être constitué de façon connue en soi par une tige, aux extrémités de laquelle sont montées coulissantes deux têtes arrondies, qu'un ressort concentrique à la tige tend à écarter l'une de l'autre, l'une de ces têtes prenant appui par sa surface arrondie contre une partie de la première branche, tandis que l'autre tête prend appui, par sa surface arrondie, contre une partie incurvée de la gâchette.

D'autres caractéristiques et avantages de l'invention apparaîtront sur les dessins annexés représentant une forme de réalisation de l'invention, dans le cas d'une articulation de genou, forme de réalisation dans laquelle le profil de came et la forme de la gâchette n'ont naturellement pas de caractère limitatif. Sur ces dessins:
La figure 1 est une vue latérale schématique, partiellement en coupe, de l'articulation en position de verrouillage, c'est-à-dire avec les deux branches de l'articulation dans une position correspondant à l'extension des montants de l'orthèse;
La figure 2 est une vue analogue de l'articulation en position déverrouillée, avec ses deux branches dans une position correspondant à la flexion des montants de l'orthèse.

L'articulation représentée sur les dessins comprend deux branches 1 et 2, articulées entre elles par un axe de rotation 3 et dans un évidement desquelles formant fourreau peut être engagée l'extrémité d'un montant d'orthèse, respectivement 4 et 5.

La partie de la branche 1 contiguë à l'axe 3 est découpée suivant un profil de came 6, avec lequel coopère une gâchette 7, logée dans un évidement 8 de la branche 2, en dehors duquel elle fait saillie vers l'extérieur, de manière à pouvoir être actionnée manuellement par le patient équipé de l'orthèse.

Cette gâchette 7 est montée pivotante autour d'un axe 13 par rapport à la branche 2 et est sollicitée par un organe de rappel élastique constitué par une tige 9, sur les extrémités de laquelle sont montées coulissantes des têtes arrondies, respectivement 10 et 11, par exemple hémisphériques, entre lesquelles est interposé un ressort 12, qui tend à les écarter l'une de l'autre. La tête arrondie 10 prend appui contre une partie évidée 14 de la paroi interne de l'évidement 8 de la branche 2, tandis que la tête 11 prend appui contre une partie incurvée 15 de la gâchette 7 tournée vers la partie 14 et disposée de façon telle que, lorsque la gâchette 7 est sollicitée manuellement, la partie incurvée 15 se déplace en rotation entre l'axe 13 et la partie 14.

Une butée 16, constituée par une goupille disposée transversalement, limite le déplacement de la gâchette 7 lorsque celle-ci est sollicitée manuellement en rotation autour de son axe 13.

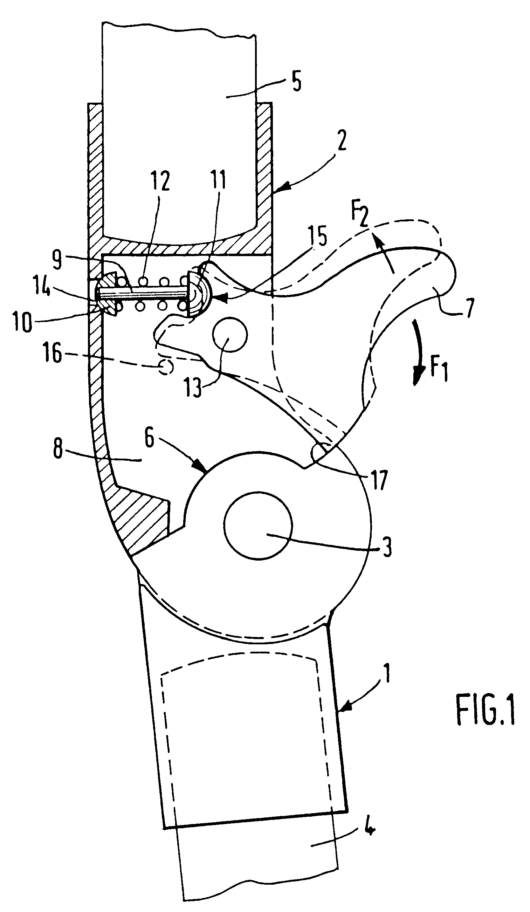

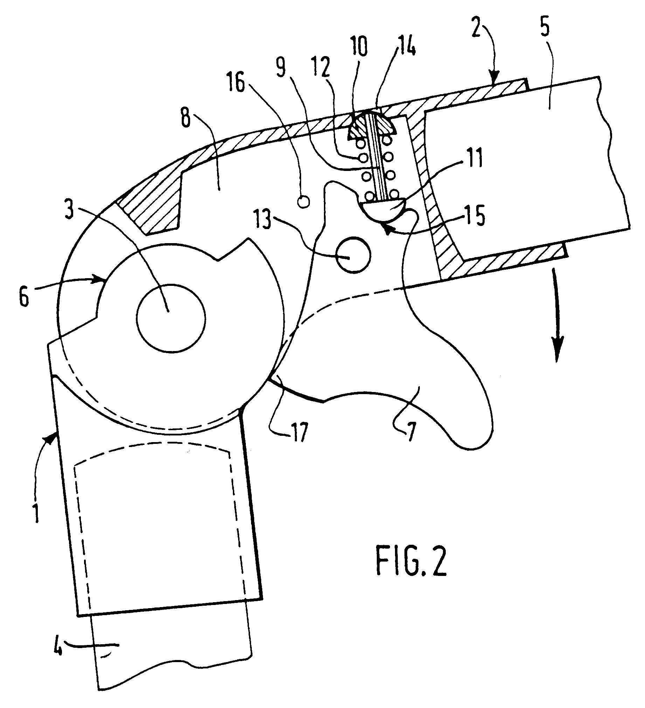

On voit ainsi que l'articulation 7 peut occuper deux positions :
- dans une première position (Figure 1), lorsque la gâchette 7 n'est pas sollicitée manuellement, son bec 17 est engagé dans le profil de came 6 et interdit à la branche de l'articulation de pivoter par rapport à la branche 2 ; dans cette position, qui correspond à la position d'extension des montants 4 et 5, la gâchette 7 est sollicitée en rotation, suivant la flèche F₁, par le système de rappel élastique comprenant de ressort 12, qui assure ainsi le verrouillage de l'articulation en cette position ;
- dans une seconde position (Figure 2), après que la gâchette 7 ait été sollicitée manuellement suivant la flèche F₂, en sens inverse de la flèche F₁, le bec 17 de la gâchette n'est plus en prise avec le profil de came 6 et les branches 1 et 2 de l'articulation peuvent pivoter librement l'une par rapport à l'autre ; dans cette position, qui correspond à la position de flexion de l'articulation, la patient équipé de l'orthèse peut ainsi s'asseoir.

Pour passer de la première à la seconde position, le patient actionne lui-même la gâchette 7 suivant la flèche F₂ à l'encontre du ressort 12, ce qui a pour effet de dégager le bec 17 de la gâchette du profil de came 6. Au cours du mouvement de la gâchette, celle-ci rencontre la butée 16 avant que le point d'appui 15 de la tête arrondie 11 ait traversé la ligne droite réunissant l'axe 13 au point 14, et le ressort continue de solliciter la gâchette suivant la flèche F₂. Lorsque la gâchette n'est plus sollicitée manuellement, elle tend donc à revenir à sa position initiale, mais avec le bec 16 en dehors du profil de came 6.

On voit que, si le patient dispose d'une certaine liberté de mouvement, il suffit d'ôter la goupille 16 formant butée, pour que, lorsque la gâchette est sollicitée manuellement, le point 15 passe à la droite de la ligne droite réunissant le point 14 et l'axe 13. L'articulation demeure alors en permanence en position déverrouillée et elle peut ainsi être utilisée comme une articulation normale.

On remarquera que, dans les conditions normales d'utilisation, avec la butée 16 en position, l'articulation revient automatiquement en position verrouillée lorsque le patient passe de la position de flexion à la position d'extension des montants 4 et 5, c'est-à-dire lorsqu'il se relève à partir d'une position assise, le bec 17 de la gâchette revenant alors s'engager lui-même dans le profil de came 6.

L'invention apporte donc un moyen simple et facile à mettre en oeuvre pour le verrouillage et le déverrouillage manuel, par le patient lui-même, d'une orthèse des membres inférieurs.

## Revendications

1. Articulation pour orthèse des membres inférieurs, à deux branches (1,2) articulées entre elles par un axe de rotation (3), comportant un système de verrouillage comprenant une gâchette (7) montée pivotante autour d'un axe (13) par rapport à une première branche (2) de l'articulation et actionnable manuellement par une partie faisant saillie à l'extérieur de cette première branche, cette gâchette (7) coopérant avec un profil (6) ménagé sur la seconde branche (1) de l'articulation et étant sollicitée par un organe de rappel élastique (12) qui prend appui, à une extrémité, contre une partie (14) de la première branche, tandis que son autre extrémité prend appui contre une partie (15) de la gâchette apte à se déplacer, le profil (6) ménagé sur la seconde branche de l'articulation est un profil de came tel que, lorsque la gâchette (7) pivote autour de son axe (13), la partie (15) de cette gâchette se déplace, entre cet axe et le point d'appui (14) de l'organe de rappel élastique sur la première branche, pour passer d'une première position dans laquelle le profil (6) interdit à la gâchette (7) non sollicitée manuellement de se déplacer et verrouille en position les deux branches de la gâchette l'une par rapport à l'autre, à une seconde position dans laquelle la gâchette sollicitée manuellement pivote autour de son axe de rotation pour échapper au profil de came (6) et venir contre une butée (16) solidaire de la première branche, les positions relatives de l'organe de butée, de l'axe de rotation de la gâchette et des points d'appui de l'organe de rappel élastique sur cette gâchette et sur la première branche étant telles que, lorsque la gâchette (7) est sollicitée manuellement, le point d'appui (15) sur la gâchette de l'organe de rappel élastique (12), au cours du mouvement de la gâchette ne traverse pas la droite réunissant l'axe (13) de rotation de la gâchette au point d'appui (14) de l'organe de rappel contre une partie de la première branche de l'articulation, de manière que la gâchette tende à revenir à sa position initiale lorsqu'elle n'est plus sollicitée manuellement, caractérisé en ce que la butée (16) est une butée amovible qui lorsqu'elle sera séparée de l'articulation permettra au point d'appui (15) sur la gâchette de l'organe de rappel élastique (12) de traverser la droite réunissant l'axe de rotation (13) de la gâchette au point d'appui (10) de l'organe de rappel contre une partie de la première branche de l'articulation.

2. Articulation selon la revendication 1, caractérisée en ce que la gâchette, son axe de rotation (13) et l'organe de rappel élastique (12) sont logés dans un évidement (8) de la première branche (2) de l'articulation, une partie de la gâchette (7) faisant saillie à l'extérieur de cet évidement (8) à travers une lumière ménagée dans la tranche de la première branche (2).

3. Articulation selon l'une des revendications 1 et 2, caractérisée en ce que,de façon connue en soi, dans la première position de la gâchette, celle-ci est en prise par un bec (17) avec le profil de came (6).

4. Articulation selon l'une des revendications 1 à 3, caractérisée en ce que, de façon connue en soi,l'organe de rappel élastique comprend une tige (9) aux extrémités de laquelle sont montées coulissante des têtes arrondies (10,11) qu'un ressort (12) tend à écarter l'une de l'autre, l'une de ces têtes (10) prenant appui par sa surface arrondie contre une partie (14) de la première branche (2), tandis que l'autre tête (11) prend appui, par sa surface arrondie, contre une partie incurvée (15) de la gâchette (7).

## Claims

1. A joint for a lower limb orthesis, with two branches (1, 2) articulated together by a rotational shaft (3), comprising a locking system comprising a tumbler (7) mounted pivotally about an axis (13) with respect to a first branch (2) of the joint and manually actuatable by a portion projecting from the exterior of this first branch, this tumbler (7) interacting with a profiled portion (6) formed on the second branch (1) of the joint and being stressed by a resilient return member (12) which rests, at one end, against part (14) of the first branch, while its other end rests against part (15) of the tumbler capable of displacement, the profiled portion (6) formed on the second branch of the joint being a cam profile such that, when the tumbler (7) pivots abouts its axis (13), the part (15) of this tumbler moves between this axis and the point (14) where the resilient return member rests on the first branch, to pass from a first position, in which the profiled portion (6) prevents the tumbler (7), which is not manually stressed, from moving and locks in position the two branches of the tumbler one with respect to the other, into a second position in which the manually stressed tumbler pivots about its rotational axis to escape the cam profile (6) and come against an abutment (16) integral with the first branch, the relative position of the abutment member, the axis of rotation of the tumbler and the points where the resilient return member rests on this tumbler and the first branch being such that, when the tumbler (7) is manually stressed, in the course of the tumbler movement the rest point (15) on the tumbler of the resilient return member (12) does not cross the straight line connecting the axis of rotation (13) of the tumbler to the rest point (14) of the return member against part of the first branch of the joint, in such a way that the tumbler tends to return to its initial position when it is no longer manually stressed, characterized in that the abutment (16) is a movable abutment which, when it is separated from the joint, permits the rest point (15) on the tumbler of the resilient return member (12) to cross the straight line connecting the axis of rotation (13) of the tumbler to the rest point (10) of the return member against part of the first branch of the joint.

2. A joint according to claim 1, characterized in that the tumbler, its axis of rotation (13) and the resilient return member (12) are accommodated in a recess (8) in the first branch (2) of the joint, part of the tumbler (7) projecting on the outside of this recess (8) through a slot formed in the edge of the first branch (2).

3. A joint according to either one of claims 1 and 2, characterized in that, in a manner known per se, when the tumbler is in its first position it is in contact with the cam profile (6) via one tip (17).

4. A joint according to any one of claims 1 to 3, characterized in that, in a manner known per se, the resilient return member comprises a rod (9) at the ends of which are slidingly mounted rounded heads (10,11) which a spring (12) tends to separate from each other, one of these heads (10) resting with its rounded surface against a portion (14) of the first branch (2), while the other head (11) rests with its rounded surface against a curved portion (15) of the tumbler (7).

## Patentansprüche

1. Orthopädisches Gelenk für untere Gliedmaßen mit zwei durch eine Drehachse (3) miteinander gelenkig verbundenen Schenkein (1,2), bestehend aus einem Verriegelungssystem mit einer Zuhaltung (7), die relativ zu einem ersten Schenkel (2) der Gelenkverbindung drehbar um eine Achse (13) angeordnet ist und durch einen von der Außenseite des ersten Schenkels vorspringenden Abschnitt von Hand betätigt werden kann, wobei die Zuhaltung (7) mit einem Profil (6) zusammenwirkt, das am zweiten Schenkel (1) des Gelenks ausgebildet ist und durch ein elastisches Rückzugelement (12) angezogen wird, das an einem Ende gegen einen Abschnitt (14) des ersten Schenkels anliegt, während das andere Ende gegen einen Abschnitt (15) der verstellbaren Zuhaltung zur Anlage kommt, wobei das auf dem zweiten Schenkel des Gelenks ausgebildete Profil (6) eine Nockenform hat dergestalt, daß sich bei Drehung der Zuhaltung (7) um ihre Achse (13) die Zuhaltung zwischen dieser Achse und dem Auflagepunkt (14) des elastischen Rückzugelements auf dem ersten Schenkel verschiebt, um aus einer ersten Stellung, in welcher das Profil (6) ohne manuelle Belastung eine Bewegung der Zuhaltung (7) verhindert und die beiden Schenkel der Zuhaltung relativ zueinander verriegelt, in eine zweite Stellung zu gelangen, in welcher die Zuhaltung sich unter manueller Belastung um ihre Drehachse schwenken läßt, um das Nockenprofil (6) zu verlassen und gegen einen Anschlag (16) zur Anlage zu kommen, der fest mit dem ersten Schenkel verbunden ist, wobei das Anschlagelement, die Drehachse der Zuhaltung und die Auflagepunkte des elastischen Rückzugelements auf der Zuhaltung und auf dem ersten Schenkel sich in solchen relativen Positionen befinden, daß bei manueller Belastung der Zuhaltung (7) der Auflagepunkt (15) auf der Zuhaltung des elastischen Rückzugelements (12) während der Bewwegung der Zuhaltung sich nicht mit der Gerade schneidet, welche die Achse (13) zum Drehen der Zuhaltung mit dem Auflagepunkt (14) des Rückzugelements gegen einen Teil des ersten Schenkels des Gelenks verbindet dergestalt, daß die Zuhaltung wieder in ihre Ausgangsstellung zurückkehrt, sobald die manuelle Belastung nicht mehr ansteht, dadurch gekennzeichnet, daß der Anschlag (16) ein lösbarer Anschlag ist, durch den sich bei Trennung des Gelenks der Auflagepunkt (15) an der Zuhaltung des elastischen Rückzugelements (12) mit der Geraden überschneiden kann, welche die Drehachse (13) der Zuhaltung mit dem Auflagepunkt (10) des Rückzugelements gegen einen Abschnitt des ersten Schenkels des Gelenks verbindet.

2. Gelenk nach Anspruch 1, dadurch gekennzeichnet, daß die Zuhaltung, deren Drehachse (13) und das elastische Rückzugelement (12) in einer Aussparung (8) des ersten Schenkels (2) des Gelenks untergebracht sind, wobei ein Teil der Zuhaltung (7) von dieser Aussparung (8) durch ein im Rand des ersten Schenkels (2) ausgebildetes Fenster nach außen vorspringt.

3. Gelenk nach irgendeinem der Ansprüche 1 und 2, dadurch gekennzeichnet, daß auf eine an sich bekannte Weise in der ersten Position der Zuhaltung diese durch eine Nase (17) mit dem Nockenprofil (6) in Eingriff steht.

4. Gelenk nach irgendeinem der Ansprüche 1 bis 3, dadurch gekennzeichnet, daß auf eine an sich bekannte Weise das elastische Rückzugelement eine Spindel (9) umfaßt, an deren Enden abgerundete Köpfe (10, 11) verschiebbar angeordnet sind, die von einer Feder (12) relativ zueinander verstellt werden, wobei einer der Köpfe (10) mit seiner abgerundeten Fläche gegen einen Abschnitt (14) des ersten Schenkels (2) anliegt, während der andere Kopf (11) mit seiner abgerundeten Fläche gegen einen nach innen gewölbten Abschnitt (15) der Zuhaltung (7) zur Anlage kommt.
